# EUROPEAN PATENT SPECIFICATION

(11) **EP 2 100 615 B1**
(45) Date of publication and mention of the grant of the patent: **10.02.2016**
(21) Application number: 09007539.1
(22) Date of filing: 14.02.2003
(51) Int. Cl.: A61K 39/00, A61P 35/00, G01N 33/68, G01N 33/574

(54) **Cancer therapy**
Krebstherapie
Thérapie de cancer

(30) Priority: 14.02.2002 AU PS054702
(43) Date of publication of application: 16.09.2009
(62) Divisional of application: 03701355.4
(73) Proprietor: ImmunAid Pty Ltd, Fitzroy, VIC 3065 (AU)
(72) Inventor: Ashdown, Martin Leonard, Victoria 3071 (AU)
(74) Representative: D Young & Co LLP

(56) References cited:
- EP-A1- 1 692 516
- EP-A1- 1 805 510
- DATABASE MEDLINE US NATIONAL LIBRARY OF MEDICINE (NLM), BETHESDA, MD, US; 1 February 1993 (1993-02-01), BATAILLE R ET AL: "[Cytokines and lymphoplasmocytic proliferations: essential role of interleukin 6]" XP002351766 & LA REVUE DU PRATICIEN. 1 FEB 1993, vol. 43, no. 3, 1 February 1993 (1993-02-01), pages 275-278, XP002351766
- DATABASE BIOSIS BIOSCIENCES INFORMATION SERVICE, PHILADELPHIA, PA, US; 1990, TONIATTI C ET AL: "REGULATION OF THE HUMAN C-REACTIVE PROTEIN GENE A MAJOR MARKER OF INFLAMMATION AND CANCER" XP002351697 & MOLECULAR BIOLOGY AND MEDICINE, vol. 7, no. 3, 1990, pages 199-212,
- LOGOTHETIS C J ET AL: "Cyclic chemotherapy with cyclophosphamide, doxorubicin, and cisplatin plus vinblastine and bleomycin in advanced germinal tumors. Results with 100 patients." THE AMERICAN JOURNAL OF MEDICINE. AUG 1986, vol. 81, no. 2, August 1986 (1986-08), pages 219-228, XP002351698 ISSN: 0002-9343
- DATABASE BIOSIS BIOSCIENCES INFORMATION SERVICE, PHILADELPHIA, PA, US; 1986, GORDON A N ET AL: "CISPLATIN VINBLASTINE AND BLEOMYCIN COMBINATION THERAPY IN RESISTANT GESTATIONAL TROPHOBLASTIC DISEASE" XP002351699 & CANCER, vol. 58, no. 7, 1986, pages 1407-1410,
- DATABASE MEDLINE US NATIONAL LIBRARY OF MEDICINE (NLM), BETHESDA, MD, US; December 1982 (1982-12), TAWA A ET AL: "[Rhabdomyosarcoma of the urinary bladder: complete remission induced by vinblastine, cis-platinum, and bleomycin]" XP002351700 & GAN TO KAGAKU RYOHO. CANCER & CHEMOTHERAPY. DEC 1982, vol. 9, no. 12, December 1982 (1982-12), pages 2222-2228,
- BERD D ET AL: "EFFECT OF LOW DOSE CYCLOPHOSPHAMIDE ON THE IMMUNE SYSTEM OF CANCER PATIENTS: DEPLETION OF CD4+, 2H4+ SUPPRESSOR-INDUCER T-CELLS" CANCER RESEARCH, AMERICAN ASSOCIATION FOR CANCER RESEARCH, BALTIMORE, MD, US, vol. 48, no. 4, 15 March 1988 (1988-03-15) , pages 1671-1675, XP000900007 ISSN: 0008-5472
- MARCHAND M ET AL: "Tumor regressions observed in patients with metastatic melanoma treated with an antigenic peptide encoded by gene MAGE-3 and presented by HLA-A1" INTERNATIONAL JOURNAL OF CANCER, NEW YORK, NY, US, vol. 80, no. 2, 18 January 1999 (1999-01-18), pages 219-230, XP002305439 ISSN: 0020-7136
- DATABASE MEDLINE US NATIONAL LIBRARY OF MEDICINE (NLM), BETHESDA, MD, US; January 1989 (1989-01), GHYKA G ET AL: "An experimental model comparing the antineoplastic and the immunosuppressive effects of some cytostatics." XP002351730 & ARCHIVES ROUMAINES DE PATHOLOGIE EXPERIMENTALES ET DE MICROBIOLOGIE. 1989 JAN-MAR, vol. 48, no. 1, January 1989 (1989-01), pages 33-45,
- ROBBERT G VAN DER MOST ET AL: "Tumor eradication after cyclophosphamide depends on concurrent depletion of regulatory T cells: a role for cycling TNFR2-expressing effector-suppressor T cells in limiting effective chemotherapy", CANCER IMMUNOLOGY, IMMUNOTHERAPY, SPRINGER, BERLIN, DE, vol. 58, no. 8, 4 December 2008 (2008-12-04), pages 1219-1228, XP019706344, ISSN: 1432-0851
- XIA WU ET AL: "The immunologic aspects in advanced ovarian cancer patients treated with paclitaxel and carboplatin chemotherapy", CANCER IMMUNOLOGY, IMMUNOTHERAPY, SPRINGER, BERLIN, DE, vol. 59, no. 2, 30 August 2009 (2009-08-30), pages 279-291, XP019757713, ISSN: 1432-0851
- SAKAGUCHI SHIMON: "Regulatory T cells: history and perspective.", METHODS IN MOLECULAR BIOLOGY (CLIFTON, N.J.) 2011, vol. 707, 2011, pages 3-17, ISSN: 1940-6029
- ZITVOGEL LAURENCE ET AL: "The anticancer immune response: indispensable for therapeutic success?", THE JOURNAL OF CLINICAL INVESTIGATION JUN 2008, vol. 118, no. 6, June 2008 (2008-06), pages 1991-2001, ISSN: 0021-9738
- MCNALLY ALICE ET AL: "CD4+CD25+ regulatory T cells control CD8+ T-cell effector differentiation by modulating IL-2 homeostasis.", PROCEEDINGS OF THE NATIONAL ACADEMY OF SCIENCES OF THE UNITED STATES OF AMERICA 3 MAY 2011, vol. 108, no. 18, 3 May 2011 (2011-05-03), pages 7529-7534, ISSN: 1091-6490
- DARRASSE-JÈZE GUILLAUME ET AL: "Tumor emergence is sensed by self-specific CD44hi memory Tregs that create a dominant tolerogenic environment for tumors in mice.", THE JOURNAL OF CLINICAL INVESTIGATION SEP 2009, vol. 119, no. 9, September 2009 (2009-09), pages 2648-2662, ISSN: 1558-8238

## Description

### FIELD OF THE INVENTION:

The present invention relates to methods for treating cancer. In particular, the present invention relates to an immunomodulation which results in the destruction of tumour cells.

### BACKGROUND OF THE INVENTION:

Although our understanding of the mechanisms and possible treatment of cancer has increased over recent years, cancer remains a major cause of death throughout the developed world. Non-specific approaches to cancer management, such as surgery, radiotherapy and generalized chemotherapy, have been successful in the management of some circulating and slow-growing solid cancers. However, many types of cancer are generally highly resistant to standard treatments. Accordingly, there is a need for further, and more effective, cancer therapies.

Immunotherapy has also been investigated as an avenue for treating cancer. The general principle of immunotherapy is to confer upon the subject being treated an ability to mount what is in effect a rejection response, specifically against the neoplastic cells. There are a number of immunological strategies under development including, adoptive immunotherapy using stimulated autologous cells of various kinds; systemic transfer of allogeneic lymphocytes; intra-tumour implantation of immunologically reactive cells; and vaccination at a distant site to generate a systemic tumour-specific immune response.

Adoptive T-cell therapy involves the passive transfer of antigen-reactive T cells to a tumour-bearing host in order to start tumour rejection. The cells are histocompatible with the subject, and are generally obtained from a previous autologous donation.

One approach to adoptive immunotherapy is to stimulate autologous lymphocytes *ex vivo* with tumour-associated antigen to make them tumour-specific. Autologous lymphocytes and killer cells may also be stimulated non-specifically. In one example, Fc receptor expressing leukocytes that can mediate an antibody-dependent cell-mediated cytotoxicity reaction are generated by culturing with a combination of IL-2 and IFN-γ (US 5,308,626). In another example, peripheral blood-derived lymphocytes cultured in IL-2 form lymphokine-activated killer (LAK) cells, which are cytolytic towards a wide range of neoplastic cells, but not normal cells. LAK are primarily derived from natural killer cells expressing the CD56 antigen, but not CD3. Such cells can be purified from peripheral blood leukocytes by IL-2-induced adherence to culture plates (A-LAK cells; see US 5,057,423).

Adoptive transfer of allogeneic lymphocytes relies on the creation of a general level of immune stimulation, and thereby overcomes the anergy that prevents the host's immune system from rejecting the tumour. Even though initial experiments were conducted over a decade ago, the strategy has not gained general acceptance, especially for the treatment of solid tumours.

Intra-tumour implantation is a strategy directed to delivering immune cells against the tumour directly to the site of action. Since the transplanted cells do not circulate, they need not be histocompatible with the host. Intratumour implantation of allogeneic cells may promote the ability of the transplanted cells to react with the tumour, and initiate a potent graft versus tumour response. For example, Kruse et al. (1990) demonstrated that direct intratumoural implantation of allogeneic cytotoxic T lymphocytes (CTL) into brain tumours growing in Fischer rats resulted in a significant survival advantage over other populations of lymphocytes, including syngeneic CTL, LAK cells, adherent-LAK cells or IL-2 alone.

Finally, the generation of an active systemic tumour-specific immune response of host origin has also been used in attempts to control cancer growth. The response is elicited from the subject's own immune system by administering a vaccine composition at a site distant from the tumour. The specific antibodies or immune cells elicited in the host as a result will hopefully migrate to the tumour, and then eradicate the cancer cells, wherever they are in the body. Various types of vaccines have been proposed, including isolated tumour-antigen vaccines and anti-idiotype vaccines. An alternative approach to an anti-tumour vaccine is to use tumour cells from the subject to be treated, or a derivative of such cells. However, in many patients tumour regression has not been detected in response to their own tumour antigen, even when comprised in a vaccine preparation.

In some approaches to increase immunogenicity, autologous or syngeneic tumour cells are genetically altered to produce a costimulatory molecule. Examples of costimulatory molecules include cell surface receptors, such as the B7-1 costimulatory molecule, allogeneic histocompatibility antigens or cytokines.

Populations of CD4+ T cells have been shown to regulate self-reactive T cells, and hence guard against autoimmune diseases (Sakaguchi et al., 2001). The inability to remove or control such self-reactive cells plays a role in many autoimmune diseases. However, the ability of the mammalian immune system to typically control such self-reactive lymphocytes limits the capability of the immune system to control tumour growth as the tumour is expressing self antigens. Many tumour antigens can be considered as autoantigens as they are often derived from the proteins of fetal development. Thereby, the immune response in the cancer patient against the cancer should be considered an autoimmune response. This response is self limiting through regulatory mechanisms.

Marchand et al., (1999) described the successful regression of metastatic melanoma in patients receiving three subcutaneous injections of the MAGE-3.A1 peptide at monthly intervals. No evidence for a cytolytic T lymphocyte (CTL) response was found in the blood of the patients who were analysed.

Despite advances in cancer therapy, particularly cancer immunotherapy, there remains a need for effective methods for treating cancer. The present invention provides an alternative immunotherapy that can be used to treat cancer.

### SUMMARY OF THE INVENTION:

The present inventor has noted that at least two populations of immune cells are produced in response to the presence of a tumour antigen. More particularly, the immune system of a mammal suffering from uncontrolled cell growth (cancer) is capable of mounting an immune response against the tumour through a group of cells herein generally referred to as "effector cells", however, a second population of cells are also produced which regulate the "effector cells", herein generally referred to as "regulator cells", limiting the mammal's ability to effectively control or eradicate the tumour.

Further, it has also been noted that the relative number of effector cells expand in response to a tumour antigen before the regulator cells expand. This provides an opportunity to prevent the production of, limit the function of, or destroy, the "regulator cells" whilst maintaining the "effector cells".

Although in some cases it is possible to screen for individuals that may be predisposed to cancer because of inherited genetic defects, the initial immune response to a tumour antigen produced by a neoplastic cell is difficult to trace as tumour growth can only be detected after the event has commenced. In fact, in many instances tumour growth is well advanced before detection.

Whilst effector cells are produced by the mammalian immune system which are capable of controlling tumour growth, their activity is compromised by the production of regulator cells which have a negative impact upon the effector cell population. In order to optimize effector cell function the present inventor has devised methods of cancer therapy that at least partially "reset" the immune response to a tumour antigen, thus providing a means to take advantage of the "effector cell" population to control tumour growth.

The present invention provides a method for determining when an agent which inhibits the production of, limits the function of, and/or destroys, regulator T cells, is to be administered to a mammalian subject with a cancer that has been exposed to a treatment which increases the number of, and/or activates, effector T cells directed against cancer cells, the method comprising analysing a sample obtained from the subject after the treatment for effector and/or regulator T cell numbers or activity, or an acute phase inflammatory marker, wherein the timing of administration of the agent will be selected such that it exerts a proportionally greater effect against the regulator T cells than the effector T cells , and wherein the agent is selected from the group consisting of anti-proliferative drugs, radiation, and an antibody which inhibits the down regulation activity of the regulator T cells.

Described herein is a method of treating cancer in a mammalian subject, the method comprising
i) reducing tumour load in the subject,
ii) allowing the number and/or activity of effector cells, directed against a tumour antigen, to increase in response to tumour antigen based stimulation of effector cells, and
iii) subsequently administering to the subject an agent which inhibits the production of, limits the function of, and/or destroys, regulator cells,
wherein the timing of administration of the agent is selected such that the activity of the effector cells is not significantly reduced.

Upon the conclusion of step i), tumour cell proliferation will recommence if any tumour cells remain, which in turn will lead to an expansion of effector cells directed against a tumour antigen(s) produced by the tumour cells.

Following the reduction in the subjects tumour load, the subject will need to be monitored to determine when the agent should be administered. Factors which can be monitored include, but are not limited to, tumour antigen levels, CD8+CD4- T cell levels, CD4+CD8- T cell levels, and acute phase inflammatory markers such as c-reactive protein levels.

Fluctuations in the levels of c-reactive protein in the subject may be monitored to determine when the agent is administered. Preferably, the agent is administered approximately when the levels of c-reactive protein begin to decrease. In this instance, effector cell production and/or activity results in increased levels of c-reactive protein. Upon clonal expansion of regulator cells, the activity of effector cells is downregulated resulting in a decrease in c-reactive protein levels.

The agent may be administered approximately when CD8+CD4- T cell numbers have peaked in response to tumour antigen based stimulation of effector cells following step i).

Since tumour antigen based stimulation following step i) results in the production of effector cells, these cells will stablize and/or result in a decrease in tumour cell numbers before regulator cells are produced. Accordingly, in a further preferred embodiment, the agent is administered approximately when the tumour cell numbers are decreasing and when regulator cell numbers are increasing. Typically, tumour cell numbers are determined by analysing the levels of an appropriate tumour cell marker.

Naturally, if surgery is conducted it will be desirable to attempt to remove all of the tumour cells. However, this is sometimes not possible due to the location of the tumour, and/or at least some tumour cells are missed. In a preferred embodiment, the tumour load is reduced in the subject by removing at least some of the cancer cells by surgery. Preferably, at least 50%, more preferably at least 75%, more preferably at least 90% and even more preferably at least 95% of the tumour cells are removed by surgery.

In an alternate preferred embodiment, the tumour load is reduced by administering an anti-cancer compound.

The anti-cancer compound can be any known molecule that either specifically or non-specifically (for example, general anti-mitotic drugs) destroys, or limit the function of, tumour cells in the subject. Preferred anti-cancer compounds include, but are not limited to, vinblastine and anhydro vinblastine.

In a further alternate preferred embodiment, the tumour load is reduced by exposing the subject to radiotherapy.

The production of effector cells could be enhanced by the administration of an antigen produced by a tumour cell of the subject.

Accordingly, the method described herein may comprise, following step i), administering an antigen produced by a tumour cell of the subject.

Also described herein is a method of treating cancer in a mammalian subject which has been exposed to anti-cancer compounds, or has had at least part of the tumour removed by surgery, or has been exposed to radiotherapy, the method comprising administering to the subject an agent which inhibits the production of, limits the function of, and/or destroys, regulator cells, wherein the timing of administration of the agent is selected such that the activity of effector cells is not significantly reduced.

Restimulation of effector cells in a mammalian subject suffering from cancer can be achieved by increasing the quantity of tumour antigen within the subject. The addition of further tumour antigens enhances the effector cell response, thereby providing an opportunity to ablate or inhibit the regulators of these new effectors.

Accordingly, described herein is a method of treating cancer in a mammalian subject, the method comprising
i) administering a tumour antigen, which results in an increase in the number of, and/or activates, effector cells directed against the tumour antigen, and
ii) subsequently administering to the subject an agent which inhibits the production of, limits the function of, and/or destroys, regulator cells,
wherein the timing of administration of the agent is selected such that the activity of the effector cells is not significantly reduced.

The agent may be administered approximately when CD8+CD4- T cell numbers have peaked in response to the administration of the tumour antigen.

In a preferred embodiment, the agent is administered approximately when the number of tumour cells has begun to stabilize or decrease following the administration of the tumour antigen.

In another preferred embodiment, the agent is administered approximately when the number of circulating tumour anitgens has begun to stabilize or decrease following the administration of the tumour antigen.

Preferably, the antigen is provided to the subject by administering a vaccine comprising the tumour antigen and a pharmaceutically acceptable carrier. More preferably, the vaccine further comprises an adjuvant.

In another embodiment the antigen is provided to the subject by administering a DNA vaccine encoding the antigen.

In yet another embodiment, the antigen is provided to the subject by the consumption of a transgenic plant expressing the antigen.

Preferably, the agent used in regulator cell ablation is selected from the group consisting of anti-proliferative drugs, radiation, and antibodies which inhibit the down regulation activity of the regulator cells. Preferably, the anti-proliferative drug is selected from the group consisting of vinblastine and anhydro vinblastine. Notably, in some instances, particularly with regard to the first aspect, the anti-cancer compound and the agent can in fact be the same molecule.

Examples of preferred antibodies include, but are not limited to, anti-CD4+, anti-CTLA-4 (cytotoxic lymphocyte-associated antigen-4), anti-GITR (glucocorticoid-induced tumour necrosis factor receptor), anti-CD28 and anti-CD25.

As would be readily appreciated by those skilled in the art, the methods of the present invention may be repeated to provide a more complete treatment.

Preferably, the mammalian subject is a human.

The present invention provides the use of an agent which inhibits the production of, limits the function of, and/or destroys, regulator T cells for the manufacture of a medicament for administering to a mammalian subject with cancer, wherein the agent will be administered subsequent to the subject's tumour load having been reduced which allows the number and/or activity of effector T cells, directed against a tumour antigen, to increase in response to tumour antigen based stimulation of effector T cells, and wherein the timing of administration of the agent will be selected such that it exerts a proportionally greater effect against the regulator T cells than the effector T cells, and wherein the agent is selected from the group consisting of anti-proliferative drugs, radiation, and an antibody which inhibits the down regulation activity of the regulator T cells.

The present invention provides the use of an anti-cancer compound for the manufacture of a medicament for administering to a mammalian subject with cancer, wherein the anti-cancer compound reduces tumour load which allows the number and/or activity of effector T cells, directed against a tumour antigen, to increase in response to tumour antigen based stimulation of effector T cells, and wherein the subject will be subsequently administered with an agent which inhibits the production of, limits the function of, and/or destroys, regulator T cells, and wherein the timing of administration of the agent will be selected such that it exerts a proportionally greater effect against the regulator T cells than the effector T cells , wherein the agent is selected from the group consisting of anti-proliferative drugs, radiation, and an antibody which inhibits the down regulation activity of the regulator T cells, and wherein the anti-cancer compound is an anti-proliferative drug or a tumour antigen.

The present invention provides an agent which inhibits the production of, limits the function of, and/or destroys, regulator T cells for use in maintaining the activity and/or production of, effector T cells in a mammalian subject to treat a cancer in the subject, characterised in that
(i) the subject's tumour load has been reduced;
(ii) the activity and/or the rate of expansion of the subject's effector T cells has peaked; and
(iii) the subject's regulator T cells are expanding,
wherein the agent is selected from the group consisting of anti-proliferative drugs, radiation, and an antibody which inhibits the down regulation activity of the regulator T cells.

Also described herein is the use of an agent which inhibits the production of, limits the function of, and/or destroys, regulator cells for the manufacture of a medicament for administering to a mammalian subject with cancer, wherein the mammalian subject has been exposed to anti-cancer compounds, or has had at least part of the tumour removed by surgery, or has been exposed to radiotherapy, and wherein the timing of administration of the agent is selected such that the activity of the effector cells is not significantly reduced.

Also described herein is the use of an agent which inhibits the production of, limits the function of, and/or destroys, regulator cells for the manufacture of a medicament for administering to a mammalian subject with cancer, wherein the subject has previously been administered with a tumour antigen which results in an increase in the number of, and/or activates, effector cells directed against the tumour antigen, and wherein the timing of administration of the agent is selected such that the activity of the effector cells is not significantly reduced.

Also described herein is the use of a tumour antigen for the manufacture of a medicament for administering to a mammalian subject with cancer producing the tumour antigen, wherein the tumour antigen which results in an increase in the number of, and/or activates, effector cells directed against the tumour antigen, and wherein the subject is subsequently administered with an agent which inhibits the production of, limits the function of, and/or destroys, regulator cells, and wherein the timing of administration of the agent is selected such that the activity of the effector cells is not significantly reduced.

In each aspect outlined above, ablation of the "regulator" population allows the "effector" population to reduce or eradicate tumour cells as any down regulation of effectors has been removed.

As will be apparent, preferred features and characteristics of one aspect of the invention are applicable to many other aspects of the invention.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

The invention is hereinafter described by way of the following non-limiting examples and with reference to the accompanying figures.

### BRIEF DESCRIPTION OF THE ACCOMPANYING DRAWINGS:

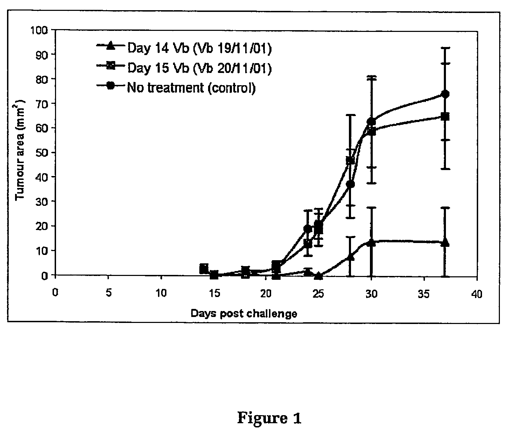

Figure 1: Effect of Vb treatment on mesothelioma area. Day 0=mesothelioma inoculation measurements with callipers at time points on day 14 and beyond. Tumour area means and standard error of the means are shown.

### DETAILED DESCRIPTION OF THE INVENTION:

### Definitions

As used herein the term "treating" or "treat" means a reduction in tumour load is achieved. In some aspects of the present invention, one step involves reducing tumour load through the use of known techniques, such as surgery, radiotherapy or chemotherapy, and then administering the agent at the appropriate time. In these aspects, the term "treating" or "treat" is used to indicate that the tumour load in the subject is further reduced by the method of the present invention. Most preferably, the tumour load is completely eradicated.

As used herein, the term "tumour load" generally refers to the number of cancerous cells in a subject at any given time.

"Regulator cells" include, but are not necessarily limited to, a subpopulation of CD4+ T cells. Such cells may also be referred to in the art as "suppressor cells". Regulator cells may either act directly on effector cells or may assert their affects upon effector cells through other mechanisms.

CD4+ cells express the marker known in the art as CD4. Typically, the term "CD4+ T cells" as used herein does not refer to cells which also express CD8. However, this term can include T cells which also express other antigenic markers such as CD25.

"Effector cells" include, but are not necessarily limited to, the T cell population known as CD8+ cells.

As used herein, the term "ablate" or "ablation" when referring to the exposure of the "regulator cells" to the agent means that the number, and/or activity, of regulator cells is down-regulated by the agent. Most preferably, the number, and/or activity, of regulator cells is completely eradicated by the agent.

As is known in the art, a cancer is generally considered as uncontrolled cell growth. The methods described herein can be used to treat any cancer including, but not limited to, carcinoma, lymphoma, blastoma, sarcoma, and leukemia. More particular examples of such cancers include breast cancer, prostate cancer, colon cancer, squamous cell cancer, small-cell lung cancer, non-small cell lung cancer, ovarian cancer, cervical cancer, gastrointestinal cancer, pancreatic cancer, glioblastoma, liver cancer, bladder cancer, hepatoma, colorectal cancer, uterine cervical cancer, endometrial carcinoma, salivary gland carcinoma, mesothelioma, kidney cancer, vulval cancer, thyroid cancer, hepatic carcinoma, skin cancer, melanoma, brain cancer, ovarian cancer, neuroblastoma, myeloma, various types of head and neck cancer, acute lymphoblastic leukemia, acute myeloid leukemia, Ewing sarcoma and peripheral neuroepithelioma.

Unless otherwise indicated, the recombinant DNA and immunological techniques utilized in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

### Agents which Inhibit the Production of, Limit the Function of, and/or Destroy, Regulator Cells

The agent can be any factor or treatment which selectively or non-selectively results in the destruction, or the inhibition of the production, of regulator cells. For example, a CD4+ specific antibody could be used to specifically target CD4+ T cells. However, in some instances a non-selective agent could be used, such as an anti-proliferative drug or radiation, both of which destroy dividing cells.

The term "anti-proliferative drug" is a term well understood in the art and refers to any compound that destroys dividing cells or inhibits them from undergoing further proliferation. Anti-proliferative drugs include, but are not limited to, mechlorethamine, cyclophosphamide, ifosfamide, melphalan, chlorambucil, hexamethyl-melamine, thiotepa, busulfan, carmustine, lomustine, semustine, streptozocin, dacarbazine, methotrexate, fluorouracil, floxuridine, cytarabine, mercaptopurine, thioguanine, pentostatin, vinblastine, anhydro vinblastine, vincristine, etoposide, teniposide, dactinomycin, daunorubicin, doxonibicin, bleomycin, plicamycin, mitomycin, L-asparaginase, cisplatin, mitoxantrone, hydroxyurea, procarbazine, mitotane, aminoglutethimide, prednisone, hydroxyprogesterone caproate, medroprogesterone acetate, megestrol acetate, diethylstilbestrol, ethinyl estradiol, tamoxifen, testosterone propionate, radioactive isotopes, ricin A chain, taxol, diphtheria toxin and pseudomonas exotoxin A.

The agent can be administered as standard dosages as used in the art. In one embodiment, the agent is administered as a single bolus injection. In another embodiment, the agent is administered by infusion over a period of, for example, 24 hours.

Recent studies have suggested that CD4+CD25+ T cells play an important role in regulating immune cells directed against self antigens (Salomon et al., 2000; Suri-Payer and Cantor, 2001). Furthermore, targeted ablation of CD4+CD25+ T cells has been shown to enhance the ability of an animal to control tumour growth (Onizuka et al., 1999; Shimizu et al., 1999; Sutmuller et al., 2001). Accordingly, CD4+CD25+ T cells could be acting as regulator cells as used herein. The activity of CD4+CD25+ T cells can be downregulated by anti-GITR, anti-CD28 and/or anti-CTLA-4 (Read et al., 2000; Takahashi et al., 2000; Shimizu et al., 2002). Thus, these antibodies may be useful as agents for use in the present invention.

### Timing of Exposing the Subject to the Agent

As outlined above, the present invention relies on the observation that the relative number of effector cells expands in response to an antigen before the regulator cells. Accordingly, as used herein, the term "the activity of the effector cells is not significantly reduced" means that the timing of the administration of the agent is such that the agent exerts a proportionally greater effect against the regulator cells than the effector cells. It is clearly preferred that the agent is administered at a time when the ratio of effect against the regulator cells to the effect against effector cells is greatest.

Some aspects of the present invention rely on reducing the tumour load before selecting for the effector cells. A number of avenues exist which enable the effector cells to be selected through ablation of the regulator cells. One comprises administering the agent approximately when CD8+CD4- T cell numbers have peaked in response to tumour antigen based stimulation of effector cells following the reduction in tumour load through techniques such as, but not limited to radiotherapy, chemotherapy and surgery. Another avenue comprises administering the agent approximately when the tumour cell numbers are decreasing and when regulator cell numbers are increasing in response to tumour antigen based stimulation of effector cells following the reduction in tumour load through techniques such as, but not limited to radiotherapy, chemotherapy and surgery.

In most instances, the time point that the agent is to be administered will need to be empirically determined in subjects at different stages of cancer as their immune response kinetics may vary. Other factors such as the general health of the subject and/or the genetic makeup of the subject will also impact upon when is the appropriate time to administer the agent.

Techniques known in the art can be used to monitor the growing population of effector cells following a reduction in the tumour load, or the stimulation of an immune response by exposing the subject to further tumour antigens. For instance, the production of acute phase proteins, such as c-reactive protein (for example as disclosed in Price et al. 1987), will indicate the presence of an immune response to the tumour antigen.

Techniques known in the art can also be used to monitor the growing population of regulator cells following a reduction in the tumour load, or the stimulation of an immune response by exposing the subject to further tumour antigens. Some of these techniques are discussed below.

Serial blood samples can be collected and quantitatively screened for all CD4+ subsets by FACS analysis. This FACS monitoring will need to be maintained until the regulator cells begin clonally expanding in response to tumour antigens, whether produced by the tumour or administered to the subject. Other possible assays for monitoring the growing population of regulator cells include lymphocyte proliferation/activation assays and various cytokine level assays (for example an assay for EL-4, JL-6 or IL- 10).

Another avenue of determining the time point for administering the agent is to monitor the tumour load. It is envisaged that the tumour load decreases due to the activity of the effector cells, however, the subsequent increase in regulator cells would down-regulate the effector cells resulting in a slowing of the tumour load decrease. Accordingly, the agent could be administered approximately prior to the slowing of the decrease in tumour load. Techniques known in the art, for example RT-PCR or antibody detection, of markers expressed by the tumour, could be used to measure tumour load in these circumstances. Examples of suitable tumour antigen marker assays include, but are not limited to, for AFP (marker for hepatocellular carcinoma and germ-cell tumours), CA 15-3 (marker for numerous cancers including breast cancer), CA 19-9 (marker for numerous cancers including pancreatic cancer and biliary tract tumours), CA 125 (marker for various cancers including ovarian cancer), calcitonin (marker for various tumours including thyroid medullary carcinoma), catecholamines and metabolites (phaeochromoctoma), CEA (marker for various cancers including colorectal cancers and other gastrointestinal cancers), hCG/beta hCG (marker for various cancers including germ-cell tumours and choriocarcinomas), 5HIAA in urine (carcinoid syndrome), PSA (prostate cancer), sertonin (carcinoid syndrome) and thyroglobulin (thyroid carcinoma).

Monitoring may need to be very frequent, for example as often as every few hours, to ensure the correct time point is selected for administration of the agent. Preferably, the monitoring is conducted at least every 48 hours. More preferably, the monitoring is conducted at least every 24 hours.

Optimally, the monitoring is continued to determine the affect of the agent. Insufficient ablation, re-emergence of the regulator cells or increases in tumour load will mean that the method described herein should be repeated. Such repeated cycles of treatment may generate immunological memory. It is therefore possible that the present invention, used in repetitive mode, may provide some prophylactic protective effet.

### C-Reactive Protein

C-reactive protein (CRP) is an important acute phase response protein, and its concentration in serum may increase as much as 1,000-fold during the acute phase response. CRP is a pentamer consisting of five identical subunits, each having a molecular weight of about 23,500.

CRP levels have previously been shown to be markers of the recurrence of a cancer (Hosotsubo et al. 2000; Mahmoud and Rivera, 2002). C-reactive protein levels can be determined using techniques known in the art, these include, but are not limited to, those disclosed in Senju et al. (1983), Price et al. (1987) and Eda et al. (1998).

### Tumour Antigens

As used herein, an "antigen" is any polypeptide sequence that contains an epitope which is capable of producing an immune response against the cancer. Preferably, the antigen will comprise a sequence which is highly selective for the cancer cell. It is envisaged that cells of a particular cancer could be characterized and an antigen produced which matches the sequences of the cancer to maximise the possibility of an effective immune response.

Antigens which are capable of raising an immune response against a cancer cell are well known in the art. Certain tumour antigens can be recognized and targeted by the immune system. This property may be due to overexpression by the tumour tissue. Some of these antigens can be detected in normal tissue. The tumour antigens targeted by T cells are generally proteins that are processed intracellularly and presented as short peptide fragments bound in the groove of the tumour MHC class I molecule to be recognized by CD8⁺ cytotoxic T lymphocytes. The mere presence of a tumour antigen is not always sufficient to trigger an immune response. Co-stimulatory molecules such as B7.1 are sometimes required. Once antigen-specific T cells are stimulated, they are capable of recognizing and destroying the tumour. The conditions needed for the activation of antigen-specific T cells are stringent, but are open to genetic manipulation of target tumour cells and T cells.

The antigen can be provided in any manner known in the art which leads to an immune response. An antigen can be, for example, native, recombinant or synthetic. Native antigens can be prepared, for example, by providing cell lysates of a tumour cell.

The antigen can be provided as isolated polypeptides in a vaccine composition. In this instance the antigen can be purified from tumour cells, expressed and isolated from recombinant cells, or synthetically produced using a peptide synthesizer.

### Vaccines

Vaccines may be prepared from one or more antigens. The preparation of vaccines which contain an antigen is known to one skilled in the art. Typically, such vaccines are prepared as injectables, or orals, either as liquid solutions or suspensions; solid forms suitable for solution in, or suspension in, liquid prior to injection or oral consumption may also be prepared. The preparation may also be emulsified, or the protein encapsulated in liposomes. The antigen is often mixed with carriers/excipients which are pharmaceutically acceptable and compatible with the active ingredient. Suitable carriers/excipients are, for example, water, saline, dextrose, glycerol, ethanol, or the like and combinations thereof

In addition, if desired, the vaccine may contain minor amounts of auxiliary substances such as wetting or emulsifying agents, pH buffering agents, and/or adjuvants which enhance the effectiveness of the vaccine.

As used herein, the term "adjuvant" means a substance that non-specifically enhances the immune response to an antigen. Examples of adjuvants which may be effective include but are not limited to: N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion. Further examples of adjuvants include aluminum hydroxide, aluminum phosphate, aluminum potassium sulfate (alum), bacterial endotoxin, lipid X, *Corynebacterium parvum* (*Propionobacterium acnes*), *Bordetella pertussis*, polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, N.J.) or Freund's Incomplete Adjuvant and Complete Adjuvant (Difco Laboratories, Detroit, Michigan).

The proportion of antigen and adjuvant can be varied over a broad range so long as both are present in effective amounts. For example, aluminum hydroxide can be present in an amount of about 0.5% of the vaccine mixture (Al₂O₃ basis). Conveniently, the vaccines are formulated to contain a final concentration of antigenic polypeptide in the range of from 0.2 to 200 µg/ml, preferably 5 to 50 µg/ml, most preferably 15 µg/ml.

After formulation, the vaccine may be incorporated into a sterile container which is then sealed and stored at a low temperature, for example 4°C, or it may be freeze-dried. Lyophilisation permits long-term storage in a stabilised form.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the vaccine composition is lyophilised, the lyophilised material may be reconstituted prior to administration, e.g. as a suspension. Reconstitution is preferably effected in buffer.

Capsules, tablets and pills for oral administration to a patient may be provided with an enteric coating comprising, for example, Eudragit "S", Eudragit "L", cellulose acetate, cellulose acetate phthalate or hydroxypropylmethyl cellulose.

### DNA Vaccination

DNA vaccination involves the direct *in vivo* introduction of DNA encoding an antigen into tissues of a subject for expression of the antigen by the cells of the subject's tissue. Such vaccines are termed herein "DNA vaccines" or "nucleic acid-based vaccines". DNA vaccines are described in US 5,939,400, US 6,110,898, WO 95/20660 and WO 93/19183. The ability of directly injected DNA that encodes an antigen to elicit a protective immune response has been demonstrated in numerous experimental systems (see, for example, Conry et al., 1994; Cardoso *et al.* 1996; Cox et al., 1993; Davis et al.,1993; Sedegah et al., 1994; Montgomery et al., 1993; Ulmer et al., 1993; Wang et al., 1993; Xiang et al., 1994; Yang et al., 1997).

To date, most DNA vaccines in mammalian systems have relied upon viral promoters derived from cytomegalovirus (CMV). These have had good efficiency in both muscle and skin inoculation in a number of mammalian species. A factor known to affect the immune response elicited by DNA immunization is the method of DNA delivery, for example, parenteral routes can yield low rates of gene transfer and produce considerable variability of gene expression (Montgomery et al., 1993). High-velocity inoculation of plasmids, using a gene-gun, enhanced the immune responses of mice (Fynan et al., 1993; Eisenbraun et al., 1993), presumably because of a greater efficiency of DNA transfection and more effective antigen presentation by dendritic cells. Vectors containing the nucleic acid-based vaccine of the invention may also be introduced into the desired host by other methods known in the art, e.g., transfection, electroporation, microinjection, transduction, cell fusion, DEAE dextran, calcium phosphate precipitation, lipofection (lysosome fusion), or a DNA vector transporter.

### Vaccines Derived from Transgenic Plants

Transgenic plants producing a antigenic polypeptide can be constructed using procedures well known in the art. A number of plant-derived edible vaccines are currently being developed for both animal and human pathogens (Hood and Jilka, 1999). Immune responses have also resulted from oral immunization with transgenic plants producing virus-like particles (VLPs), or chimeric plant viruses displaying antigenic epitopes (Mason *et al*., 1996; Modelska *et al*., 1998; Kapustra *et al*., 1999; Brennan *et al*., 1999). It has been suggested that the particulate form of these VLPs or chimeric viruses may result in greater stability of the antigen in the stomach, effectively increasing the amount of antigen available for uptake in the gut (Mason *et al.* 1996, Modelska *et al.* 1998).

### EXAMPLES:

### Example 1

Malignant mesothelioma in humans is an asbestos induced, incurable tumour that has a particularly poor prognosis. Average time from diagnosis to death being only eight months (Musk and Woodward, 1982). A murine model was developed by researchers in the Department of Medicine at the University of Western Australia to further the development of suitable interventions (Davies *et al.*, 1992). The murine tumours exhibit morphological features, distributions, and mode of spread in body cavities consistent with the human clinical situation.

Day 0 was defined as the day of tumour cell inoculation with 1x10⁶ AE 17 cells subcutaneously. The AE 17 tumour cell line was derived from C57/B16 mice and inoculated back into the same strain. This point will be referred to as a tumour challenge henceforth. Three groups of 5 mice were all challenged in this way. Subsequently the first of the groups was given a single ip dose of vinblastine (Vb) at 6mg/kg body weight exactly 14 days post tumour challenge (Vb 19/11/01 group). The second group of 5 mice were similarly treated the next day; 15 days post tumour challenge (Vb 20/11/01). The third group received no Vb treatment (No treatment/control group).

Tumour measurements were taken from Day 14 onwards with callipers (2 diameters at right angles to each other in mm's). These two diameters are then multiplied together to give a mm² value that is known from past experience to correlate well with both tumour volume and tumour mass. The resultant data from this experiment is presented in Table 1 and Figure 1.

Clearly the mice receiving the day 14 post challenge single Vb treatment are heavily protected from tumour development. In contrast the control and day 15 Vb treatment groups show significant tumour development in 4/5 mice. The data from this shows that a narrow "therapeutic window" for Vb treatment was shown and an 80% effectiveness at this time point.

**Table 1.**

| Treatment | Animal # | 19-Nov 14* | 20-Nov 15* | 23-Nov 18* | 26-Nov 21* | 29-Nov 24* | 30-Nov 25* | 3-Dec 28* | 5-Dec 30* | 7-Dec 37* |
|---|---|---|---|---|---|---|---|---|---|---|
| Vb 19/11/01 | 1 | 4 | 0.5 | 0 | 0.5 | 0 | 0 | 0 | 0 | 0 |
| Day 14 | 2 | 4 | 0.5 | 2 | 0.5 | 6 | 1 | 0.5 | 0 | 0 |
| | 3 | 4 | 0.5 | 1 | 1 | 4 | 0.5 | 40 | 70 | 70 |
| | 4 | 4 | 0 | 0 | 0 | 0 | 0 | 0 | 0 | 0 |
| | 5 | 0.5 | 0 | 0.5 | 0 | 0 | 0 | 0 | 0 | 0 |
| | | 3.3 | 0.3 | 0.7 | 0.4 | 2 | 0.3 | 8.1 | 14 | 14 |
| Vb 20/11/01 | 1 | 0.5 | 1 | 4 | 9 | 16 | 20 | 42 | 56 | 81 |
| Day 15 | 2 | 0.5 | 0.5 | 0.5 | 0.5 | | | | | |
| | 3 | 0.5 | 0 | 0 | 0.5 | 0.5 | 0.5 | 0.5 | 0.5 | 0.5 |
| | 4 | 4 | 0.5 | 0.5 | 8 | 2.5 | 30 | 56 | 90 | 90 |
| | 5 | 9 | 0.5 | 0 | 0.5 | 12 | 25 | 90 | 90 | 90 |
| | | 2.9 | 0.5 | 1 | 3.7 | 13.4 | 18.9 | 47.1 | 59.1 | 65.4 |
| No treatment | 1 | 0.5 | 0.5 | 0.5 | 1 | 30 | 20 | 42 | 99 | 99 |
| Control | 2 | 0.5 | 0 | 0 | 2 | 6 | 20 | 20 | 42 | 100 |
| | 3 | 4 | 0 | 0.5 | 0 | 0 | 0 | 0 | 0 | 0 |
| | 4 | 4 | 0.5 | 1 | 4 | 20 | 30 | 42 | 90 | 90 |
| | 5 | 4 | 1 | 9 | 12 | 40 | 36 | 84 | 84 | 84 |
| | | 2.6 | 0.4 | 2.2 | 3.8 | 19.2 | 21.2 | 37.6 | 63 | 74.6 |

### Example 2

A prostate cancer patient with elevated levels of PSA associated with the cancer is subjected to a standard chemotherapy regime involving cyclophosphamide (for example, as described by Casciato and Lowitz, 1995). Upon completion of the chemotherapy the patient is monitored for the PSA marker. Such monitoring should preferably occur at least every 24 hours after the completion of chemotherapy and continue for at least one month.

If PSA marker levels begin to elevate it will be clear that the chemotherapy is not completely successful. PSA levels are continued to be monitored until levels of PSA are decreasing. This indicates that the effector cells are beginning to control the new cancerous cells. At approximately this point vinblastine is administered to the patient at a standard dose such as 3-4 mg/m² intravenously (Casciato and Lowitz, 1995). Vinblastine will target dividing cells, such as the regulator cells, which begin to clonally expand to control effector cell levels.

The patient is further monitored for PSA levels to ensure that the cancer is controlled. Treatment is repeated if necessary.

### Example 3

A breast cancer patient with elevated levels of CA 15-3 (epitope of Polymorphic Epithelial Mucin) associated with the cancer is subjected to surgery in an attempt to remove the tumour. Upon completion of the chemotherapy the patient is monitored for the CA 15-3 marker. Such monitoring should preferably occur at least every 24 hours after surgery and continue for at least one month.

If CA 15-3 marker levels begin to elevate it is clear that all of the tumour cells are not removed by the surgery. CA 15-3 levels are continued to be monitored until levels of CA 15-3 are decreasing. This indicates that the effector cells are beginning to control the new cancerous cells. The patient is then subjected to radiation therapy to target dividing cells, for example provided as megavoltage gamma irradiation to the entire breast (about 4500-5000 cGy) (Casciato and Lowitz, 1995).

The patient is further monitored for CA 15-3 levels to ensure that the cancer is controlled. Treatment is repeated if necessary.

### Example 4

An acute lymphoblastic leukemia patient is subjected to a standard chemotherapy regime involving vincristine and prednisone (Casciato and Lowitz, 1995). Upon completion of the chemotherapy the patient is monitored for c-reactive protein levels as generally described by Price et al. (1987). Such monitoring should preferably occur at least every 24 hours after the completion of chemotherapy and continue for at least one month.

If c-reactive protein levels begin to elevate it will indicate that the patients immune system is mounting an immune response to an antigen, and that all cancerous cells are not eliminated by the chemotherapy. Naturally, the patient should be examined for any indications of, for example, viral or bacterial infections which may contribute to the elevated c-reactive protein levels. In the absence of such indications, c-reactive protein levels are continued to be monitored until levels of c-reactive protein peak and begin to decrease. This indicates that the effector cells are beginning to control the new cancerous cells. At this point anti-CD4+ antibodies which at least target part of the regulator cell population are administered to the patient at a standard doses such as 300mg.

The patient is further monitored for c-reactive protein levels to ensure that the cancer is controlled. Treatment repeated if necessary.

### Example 5

A melanoma patient is examined for MAGE-3 which is expressed in approximately 76% of melanoma patients. If MAGE-3 expression is detected the patient is injected with a suitable MAGE-3 peptide in a vaccine composition as generally described by Coulie et al. (2001).

The patient is then monitored for CD8+CD4- T cell numbers, preferably at least every 24 hours for at least one month. Approximately when CD8+CD4- T cell numbers have peaked in response to tumour antigen based stimulation of effector cells the patient is administered with vinblastine at a standard dose such as 3-4 mg/m² intravenously. Vinblastine will target dividing cells, such as regulator cells which clonally expand to control effector cell levels.

The patient is further monitored for cancerous cells, such as using the S100 protein as a marker (Casciato and Lowitz, 1995). If further melanoma cells are detected the treatment can be repeated.

As the skilled addressee would be aware, the general methods used to reduce tumour load, target regulator cells, and determine the timing of administering the agent are typically interchangeable for any given treatment. Naturally, standard techniques of, for example chemotherapy, radiation therapy, surgery and tumour cell marker detection, will preferably be performed as already used in the art (for example see (Casciato and Lowitz, 1995).

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this application.

### REFERENCES:

Brennan, F.R, Bellaby, T., Helliwell, S.M., Jones, T.D., Kamstrup, S., Dalsgaard, K., Flock, J-I. and Hamilton, W.D.O. (1999) J. Virol. 73,930-938.
Cardoso, A.I., Blixenkrone-Moller, M, Fayolle, J., Liu, M., Buckland, R and Wild, T.F. (1996) Virology 225,293-299.
Casciato, D.A. and Lowitz, B.B. (1995) Manual of Clinical Oncology. Third Edition. Little Brown and Company, Boston.
Conry, R.M., LoBuglio, A.F., Kantor, J., Schlom, J., Loechel, F., Moore, S.E., Sumerel, L.A, Barlow, D.L., Abrams, S. and Curiel, D.T. (1994) Cancer Res. 54,1164-1168.
Coulie, P.G., Karanikas, V., Colau, D., Lurquin, C., Landry, C., Marchand, M, Dorval, T., Brichard, V. and Boon, T. (2001) Proc. Natl. Acad. Sci., USA 98, 10290-10295.
Cox, G.J., Zamb, T.J. and Babiuk, L.A (1993) J. Virol. 67,5664-5667.
Davis, H.L., Michel, M.L. and Whalen, R.G. (1993) Human Mol. Genet. 2,1847-1851.
Davies, M.R., Manning, L.S., Whitaker, D., Garlepp, M.J. and Robinson, B.W. (1992). Int. J. Cancer 52,881-886.
Eda, S., Kaufmann, J., Roos, W. and Phol, S. (1998). J. Clin. Lab. Anal. 12,137-144.
Eisenbraun, M.D., Fuller, D.H. and Haynes, J.R. (1993) DNA Cell Biol. 12,791-797.
Fynan, E.F., Webster, R.G., Fuller, D.H., Haynes, J.R, Santoro, J.C. and Robinson, H.L. (1993) Proc. Natl. Acad. Sci. U.S.A. 90,11478-11482.
Hood, E.E. and Jilka, J.M. (1999) Current Opin. Biotech. 10,382-386.
Hosotsubo, K.K., Nishimura, M. and Nishimura, S. (2000) Crit. Care 4,180-187.
Kapustra, J., Modelska, A., Figlerowicz, M., Pniewski, T., Letellier, M., Lisowa, O., Yusibov, V., Koprowski, H., Plucienniczak, A. and Legocki, A.B. (1999) FASEB J. 13,1796-1799.
Kruse, C.A., Lillehei, K.O., Mitchell, D.H., Kleinschmidt-DeMasters, B. and Bellgrau, D. (1990) Proc. Natl. Acad. Sci. U.S.A. 87,9577-9581.
Mahmoud, F.A. and Rivera, N.I. (2002) Curr. Oncol. Rep. 4,250-255.
Marchand, M., Van Baren, N., Weynants, P., Brichard, V., Dreno, B., Tessier, M-H., Rankin, E., Parmiani, G., Arienti, F., Humblet, Y., Bourlond, A., Vanwijck, R., Lienard, D., Beauduin, M., Dietrich, P-Y., Russo, V., Kerger, J., Masucci, G., Elke, Jäger., De Greve, J., Atzpodien, J., Brasseur, F., Coulie, P.G., Van Der Bruggen, P. and Boon, T. (1999) Int. J. Cancer 80,219-230.
Mason H.S., Ball J.M., Shi J.J., Jiang X., Estes M.K. and Arntzen C.J. (1996) Proc. Natl. Acad. Sci. USA 93,5335-5340.
Modelska, A., Dietzschold, B., Sleysh, N., Fu, Z.F., Steplewski, K., Hooper, D.C., Koprowski, H. and Yusibov, V. (1998) Proc. Natl Acad. Sci. U.S.A. 95,2481-2485.
Montgomery, D.L., Shiver, J.W., Lender, K.R., Perry, H.C., Friedman, A., Martinez, D., Ulmer, J.B., Donnelly, J.J. and Lui, M.A. (1993) DNA Cell Biol, 12,777-783.
Musk, A.W. and Woodward, S.D. (1982) Aust. NZ J. Med. 12,229-232.
Onizuka, S., Tawara, I., Shimizu, J., Sakaguchi, S., Fujita, T. and Nakayama, E. (1999) Cancer Res. 59,3128-3133.
Price, C.P., Trull, A.K., Berry, D. and German, E.G. (1987) J. Immunol. Methods 99,205-211,
Read, S., Malmstrom, V. and Powrie, F. (2000) J. Exp. Med. 192,295-302.
Sakaguchi, S., Sakaguchi, N., Shimizu, J., Yamazaki, S., Sakihama, T., Itoh, M., Kuniyasu, Y., Nomura, T., Toda, M. and Takahashi, T. (2001) immunological Reviews 182, 18-32.
Salomon, B., Lenschow, D. J., Rhee, L., Ashourian, N., Singh, B., Sharpe, A. and Bluestone, J. A. (2000) Immunity 12,431-440.
Sedegah, M., Hedstrom, R, Hobart, P. and Hoffman, S.L. (1994) Proc. Natl Acad. Sci. U.S.A. 91,9866-9870.
Senju, O., Takagi, Y., Gomi, K., Ishii, N., Mochizuki, S. Ishii, N. et al. (1983) Jap. J. Clin. Lab. Automat. 8,161-165.
Shimizu, J., Yamazaki, S. and Sakaguchi, S. (1999) J. Immunol. 163,5211-5218.
Shimiszu, J., Yamazaki, S., Takahashi, T., Ishida, Y. and Sakaguchi, S. (2002) Nature Immunol. 3,135-142.
Suri-Payer, E. and Cantor, H. (2001) J. Autoimmunity 16,115-23.
Sutmuller, R. P., van Duivenvoorde, L. M., van Elsas, A, Schumacher, T. N., Wildenberg, M. E., Allison, J. P., Toes, R. E., Offringa, R. and Melief, C. J. (2001) J. Exp. Med. 194,823-832.
Takahashi, T., Tagami, T., Yamazaki, S., Uede, T., Shimizu, J., Sakaguchi, N., Mak, T. W. and Sakaguchi, S. (2000) J. Exp. Med. 192,303-310.
Ulmer, J.B., Donnelly, J.J., Parker, S.E., Rhodes, G.H., Felgner, P.L., Dwarki, V.J., Gromkowski, S.H., Deck, R.R., DeWitt, C.M., Friedman, A. et al. (1993) Science 259,1745-1749.
Wang, B., Ugen, K.E., Srikantan, V., Agadjanyan, M.G., Dang, K., Refaeli, Y., Sato, A.I., Boyer, J., Williams, W.V. and Weiner, D.B. (1993) Proc. Natl. Acad. Sci. U.S.A. 90,4156-4160.
Xiang, Z.Q., Spitalnik, S., Tran, M., Wunner, W.H., Cheng, J. and Ertl, H.C. (1994) Virology 199,132-140.
Yang, K., Mustafa, F., Valsamakis, A., Santoro, J.C., Griffin, D.E. and Robinson, H.L. (1997) Vaccine 15,888-891.

The invention also relates to the following aspects as defined in the following numbered paragraphs:
1. A method of treating cancer in a mammalian subject, the method comprising
   i) reducing tumour load in the subject,
   ii) allowing the number and/or activity of effector cells, directed against a tumour antigen, to increase in response to tumour antigen based stimulation of effector cells, and
   iii) subsequently administering to the subject an agent which inhibits the production of, limits the function of, and/or destroys, regulator cells,
      wherein the timing of administration of the agent is selected such that the activity of the effector cells is not significantly reduced.
2. The method of paragraph 1, wherein fluctuations in the levels of c-reactive protein in the subject are monitored to determine when the agent is administered.
3. The method of paragraph 2, wherein the agent is administered when the levels of c-reactive protein begin to decrease.
4. The method of paragraph 1, wherein the agent is administered approximately when CD8+CD4- T cell numbers have peaked in response to tumour antigen based stimulation of effector cells following step i).
5. The method of paragraph 1, wherein the agent is administered approximately when the tumour cell numbers are decreasing and when regulator cell numbers are increasing.
6. The method according to any one of paragraphs 1 to 5, wherein tumour load is reduced in the subject by removing at least some of the cancer cells by surgery.
7. The method according to any one of paragraphs 1 to 5, wherein the tumour load is reduced by administering an anti-cancer compound.
8. The method of paragraph 7, wherein the anti-cancer compound is vinblastine or anhydro vinblastine.
9. The method according to any one of paragraphs 1 to 5, wherein the tumour load is reduced by exposing the subject to radiotherapy.
10. The method according to any one of paragraphs 1 to 9, wherein the method further comprises, following step i), administering an antigen produced by a tumour cell of the subject.
11. A method of treating cancer in a mammalian subject which has been exposed to anti-cancer compounds, or has had at least part of the tumour removed by surgery, or has been exposed to radiotherapy, the method comprising administering to the subject an agent which inhibits the production of, limits the function of, and/or destroys, regulator cells, wherein the timing of administration of the agent is selected such that the activity of effector cells is not significantly reduced.
12. A method of treating cancer in a mammalian subject, the method comprising
   i) administering a tumour antigen, which results in an increase in the number of, and/or activates, effector cells directed against the tumour antigen, and
   ii) subsequently administering to the subject an agent which inhibits the production of, limits the function of, and/or destroys, regulator cells,
      wherein the timing of administration of the agent is selected such that the activity of the effector cells is not significantly reduced.
13. The method of paragraph 12, wherein fluctuations in the levels of c-reactive protein in the subject are monitored to determine when the agent is administered.
14. The method of paragraph 13, wherein the agent is administered when the levels of c-reactive protein begin to decrease.
15. The method of paragraph 12, wherein the agent is administered approximately when CD8+CD4- T cell numbers have peaked in response to the administration of the tumour antigen.
16. The method of paragraph 12, wherein the agent is administered approximately when the number of tumour cells has begun to stabilize or decrease following the administration of the tumour antigen.
17. The method of paragraph 12, wherein the agent is administered approximately when the number of circulating tumour antigens has begun to stabilize or decrease following the administration of the tumour antigen.
18. The method according to any one of paragraphs 12 to 17, wherein the antigen is provided to the subject by administering a vaccine composition comprising the tumour antigen and a pharmaceutically acceptable carrier.
19. The method of paragraph 18, wherein the vaccine composition further comprises an adjuvant.
20. The method according to any one of paragraphs 12 to 17, wherein the antigen is provided to the subject by administering a DNA vaccine encoding the antigen.
21. The method according to any one of paragraphs 12 to 17, wherein the antigen is provided to the subject by the consumption of a transgenic plant expressing the antigen.
22. The method according to any one of paragraphs 1 to 21, wherein the agent is selected from the group consisting of anti-proliferative drugs, radiation, and an antibody which inhibit the down regulation activity of the regulator cells.
23. The method of paragraph 22, wherein the antibody is anti-CD4+.
24. The method according to any one of paragraphs 1 to 23, wherein the method is repeated at least once.
25. The method according to any one of paragraphs 1 to 24, wherein the mammalian subject is a human.
26. Use of an agent which inhibits the production of, limits the function of, and/or destroys, regulator cells for the manufacture of a medicament for administering to a mammalian subject with cancer, wherein the agent is administered subsequent to the subject's tumour load having been reduced which allows the number and/or activity of effector cells, directed against a tumour antigen, to increase in response to tumour antigen based stimulation of effector cells, and wherein the timing of administration of the agent is selected such that the activity of the effector cells is not significantly reduced.
27. Use of an anti-cancer compound for the manufacture of a medicament for administering to a mammalian subject with cancer, wherein the anti-cancer compound reduces tumour load which allows the number and/or activity of effector cells, directed against a tumour antigen, to increase in response to tumour antigen based stimulation of effector cells, and wherein the subject is subsequently administered with an agent which inhibits the production of, and/or destroys, regulator cells, and wherein the timing of administration of the agent is selected such that the activity of the effector cells is not significantly reduced.
28. Use of an agent which inhibits the production of, limits the function of, and/or destroys, regulator cells for the manufacture of a medicament for administering to a mammalian subject with cancer, wherein the mammalian subject has been exposed to anti-cancer compounds, or has had at least part of the tumour removed by surgery, or has been exposed to radiotherapy, and wherein the timing of administration of the agent is selected such that the activity of the effector cells is not significantly reduced.
29. Use of an agent which inhibits the production of, limits the function of, and/or destroys, regulator cells for the manufacture of a medicament for administering to a mammalian subject with cancer, wherein the subject has previously been administered with a tumour antigen which results in an increase in the number of, and/or activates, effector cells directed against the tumour antigen, and wherein the timing of administration of the agent is selected such that the activity of the effector cells is not significantly reduced.
30. Use of a tumour antigen for the manufacture of a medicament for administering to a mammalian subject with cancer producing the tumour antigen, wherein the tumour antigen which results in an increase in the number of, and/or activates, effector cells directed against the tumour antigen, and wherein the subject is subsequently administered with an agent which inhibits the production of, limits the function of, and/or destroys, regulator cells, and wherein the timing of administration of the agent is selected such that the activity of the effector cells is not significantly reduced.

## Claims

1. A method for determining when an agent which inhibits the production of, limits the function of, and/or destroys, regulator T cells, is to be administered to a mammalian subject with a cancer that has been exposed to a treatment which increases the number of, and/or activates, effector T cells directed against cancer cells, the method comprising analysing a sample obtained from the subject after the treatment for effector and/or regulator T cell numbers or activity, or an acute phase inflammatory marker, wherein the timing of administration of the agent will be selected such that it exerts a proportionally greater effect against the regulator T cells than the effector T cells , and wherein the agent is selected from the group consisting of anti-proliferative drugs, radiation, and an antibody which inhibits the down regulation activity of the regulator T cells.

2. The method of claim 1, wherein the method includes
i) determining CD8+CD4- T cell numbers, and/or
ii) determining tumour cell numbers.

3. The method of claim 1 or claim 2, wherein the method comprises analysing samples obtained about at least every 48 hours, or at least every 24 hours, from the subject after the increase in the number or activity of effector T cells.

4. Use of an agent which inhibits the production of, limits the function of, and/or destroys, regulator T cells for the manufacture of a medicament for administering to a mammalian subject with cancer, wherein the agent will be administered subsequent to the subject's tumour load having been reduced which allows the number and/or activity of effector T cells, directed against a tumour antigen, to increase in response to tumour antigen based stimulation of effector T cells, and wherein the timing of administration of the agent will be selected such that it exerts a proportionally greater effect against the regulator T cells than the effector T cells, and wherein the agent is selected from the group consisting of anti-proliferative drugs, radiation, and an antibody which inhibits the down regulation activity of the regulator T cells.

5. Use of an anti-cancer compound for the manufacture of a medicament for administering to a mammalian subject with cancer, wherein the anti-cancer compound reduces tumour load which allows the number and/or activity of effector T cells, directed against a tumour antigen, to increase in response to tumour antigen based stimulation of effector T cells, and wherein the subject will be subsequently administered with an agent which inhibits the production of, limits the function of, and/or destroys, regulator T cells, and wherein the timing of administration of the agent will be selected such that it exerts a proportionally greater effect against the regulator T cells than the effector T cells , wherein the agent is selected from the group consisting of anti-proliferative drugs, radiation, and an antibody which inhibits the down regulation activity of the regulator T cells, and wherein the anti-cancer compound is an anti-proliferative drug or a tumour antigen.

6. The use of claim 4 or claim 5, wherein fluctuations in the levels of an acute phase inflammatory marker in the subject will be monitored to determine when the agent will be administered.

7. The use according to any one of claims 4 to 6, wherein the agent will be administered approximately when
i) the number of tumour cells has begun to stabilize or decrease following the administration of the tumour antigen,
ii) the number of circulating tumour antigens has begun to stabilize or decrease following the administration of the tumour antigen, and/or
iii) the tumour cell numbers are decreasing and when regulator cell numbers are increasing.

8. The use according to any one of claims 4 to 7, wherein the mammalian subject is a human.

9. An agent which inhibits the production of, limits the function of, and/or destroys, regulator T cells for use in maintaining the activity and/or production of effector T cells in a mammalian subject to treat a cancer in the subject, **characterised in that**
(i) the subject's tumour load has been reduced;
(ii) the activity and/or the rate of expansion of the subject's effector T cells has peaked; and
(iii) the subject's regulator T cells are expanding,
wherein the agent is selected from the group consisting of anti-proliferative drugs, radiation, and an antibody which inhibits the down regulation activity of the regulator T cells.

10. The agent for use according to claim 9 wherein the subject's effector cells are not expanding.

11. The agent for use according to claim 9 or claim 10 wherein the subject's tumour cells are decreasing.

12. The use according to any one of claims 4 to 8 or the agent for use according to any one of claims 9 to 11, wherein the agent is mechlorethamine, cyclophosphamide, ifosfamide, melphalan, chlorambucil, hexamethyl-melamine, thiotepa, busulfan, carmustine, lomustine, semustine, streptozocin, dacarbazine, methotrexate, fluorouracil, floxuridine, cytarabine, mercaptopurine, thioguanine, pentostatin, vinblastine, anhydro vinblastine, vincristine, etoposide, teniposide, dactinomycin, daunorubicin, doxorubicin, bleomycin, plicamycin, mitomycin, L-asparaginase, cisplatin, mitoxantrone, hydroxyurea, procarbazine, mitotane, aminoglutethimide, prednisone, hydroxyprogesterone caproate, medroprogesterone acetate, megestrol acetate, diethylstilbestrol, ethinyl estradiol, tamoxifen, testosterone propionate, radioactive isotopes, ricin A chain, taxol, diphtheria toxin, pseudomonas exotoxin A, an anti-CD4 antibody, an anti-CTLA-4 antibody, an anti-GITR antibody, an anti-CD28 antibody, or an anti CD25 antibody.

13. The use or the agent for use according to claim 12 wherein the agent is vinblastine or anhydro vinblastine.

## Patentansprüche

1. Verfahren zur Feststellung, wann ein Mittel, das die Produktion von regulatorischen T-Zellen inhibiert, deren Funktion einschränkt und/oder diese zerstört, einem Säugetier-Patienten mit einer Krebserkrankung, die einer Behandlung, die die Anzahl von gegen Krebszellen gerichteten Effektor-T-Zellen erhöht und/oder diese aktiviert, ausgesetzt wurde, zu verabreichen ist, wobei das Verfahren die Analyse einer von dem Patienten nach der die Anzahl bzw. Aktivität von Effektor- und/oder regulatorischen T-Zellen betreffenden Behandlung erhaltenen Probe oder eines Markers für die akute Phase einer Entzündung umfasst, wobei der Zeitpunkt der Verabreichung des Mittels so gewählt wird, dass es eine proportional größere Wirkung gegen die regulatorischen T-Zellen als gegen die Effektor-T-Zellen ausübt, und wobei das Mittel aus der aus antiproliferativen Arzneimitteln, Strahlung und einem Antikörper, der die Herunterregulierungsaktivität der regulatorischen T-Zellen inhibiert, bestehenden Gruppe ausgewählt ist.

2. Verfahren nach Anspruch 1, wobei das Verfahren
i) die Bestimmung der CD8+CD4-T-Zellen-Zahlen und/oder
ii) die Bestimmung der Tumorzellenzahlen umfasst.

3. Verfahren nach Anspruch 1 oder Anspruch 2, wobei das Verfahren die Analyse von Proben umfasst, die etwa mindestens alle 48 Stunden oder mindestens alle 24 Stunden nach der Zunahme der Anzahl oder der Aktivität von Effektor-T-Zellen von dem Patienten erhalten wurden.

4. Verwendung eines Mittels, das die Produktion von regulatorischen T-Zellen inhibiert, deren Funktion einschränkt und/oder diese zerstört, zur Herstellung eines Medikaments zur Verabreichung an einen Säugetier-Patienten mit Krebs, wobei das Mittel im Anschluss an die Reduktion der Tumorlast des Patienten verabreicht wird, wodurch die Anzahl und/oder Aktivität von gegen ein Tumorantigen gerichteten Effektor-T-Zellen als Reaktion auf eine tumorantigenbasierte Stimulierung von Effektor-T-Zellen zunehmen kann und wobei der Zeitpunkt der Verabreichung des Mittels so gewählt wird, dass es eine proportional größere Wirkung gegen die regulatorischen T-Zellen als gegen die Effektor-T-Zellen ausübt, und wobei das Mittel aus der aus antiproliferativen Arzneimitteln, Strahlung und einem Antikörper, der die Herunterregulierungsaktivität der regulatorischen T-Zellen inhibiert, bestehenden Gruppe ausgewählt ist.

5. Verwendung einer Antikrebsverbindung zur Herstellung eines Medikaments zur Verabreichung an einen Säugetier-Patienten mit Krebs, wobei die Antikrebsverbindung die Tumorlast reduziert, wodurch die Anzahl und/oder Aktivität von gegen ein Tumorantigen gerichteten Effektor-T-Zellen als Reaktion auf eine tumorantigenbasierte Stimulierung von Effektor-T-Zellen zunehmen kann und wobei dem Patienten anschließend ein Mittel, das die Produktion von regulatorischen T-Zellen inhibiert, deren Funktion einschränkt und/oder diese zerstört, verabreicht wird und wobei der Zeitpunkt der Verabreichung des Mittels so gewählt wird, dass es eine proportional größere Wirkung gegen die regulatorischen T-Zellen als gegen die Effektor-T-Zellen ausübt, und wobei das Mittel aus der aus antiproliferativen Arzneimitteln, Strahlung und einem Antikörper, der die Herunterregulierungsaktivität der regulatorischen T-Zellen inhibiert, bestehenden Gruppe ausgewählt ist und wobei es sich bei der Antriebsverbindung um ein antiproliferatives Arzneimittel ode rein Tumorautigen handelt.

6. Verwendung nach Anspruch 4 oder Anspruch 5, wobei Schwankungen bei den Spiegeln eines Markers für die akute Phase einer Entzündung in dem Patienten kontrolliert werden um festzustellen, wann das Mittel verabreicht wird.

7. Verwendung nach einem der Ansprüche 4 bis 6, wobei das Mittel ungefähr dann verabreicht wird, wenn
i) die Anzahl der Tumorzellen sich nach der Verabreichung des Tumorantigens zu stabilisieren oder abzunehmen begonnen hat,
ii) die Anzahl der im Blutkreislauf befindlichen Tumorantigene sich nach der Verabreichung des Tumorantigens zu stabilisieren oder abzunehmen begonnen hat und/oder
iii) die Anzahl der Tumorzellen abnimmt und die Anzahl der regulatorischen Zellen zunimmt.

8. Verwendung nach einem der Ansprüche 4 bis 7, wobei es sich bei dem Säugetier-Patienten um einen Menschen handelt.

9. Mittel, das die Produktion von regulatorischen T-Zellen inhibiert, deren Funktion einschränkt und/oder diese zerstört, zur Verwendung bei der Aufrechterhaltung der Aktivität und/oder Produktion von Effektor-T-Zellen in einem Säugetier-Patienten zur Behandlung einer Krebserkrankung in dem Patienten, **dadurch gekennzeichnet, dass**
(i) die Tumorlast des Patienten reduziert wurde,
(ii) die Aktivität und/oder die Expansionsrate der Effektor-T-Zellen des Patienten einen Spitzenwert überschritten hat und
(iii) die regulatorischen T-Zellen des Patienten expandieren,
wobei das Mittel aus der aus antiproliferativen Arzneimitteln, Strahlung und einem Antikörper, der die Herunterregulierungsaktivität der regulatorischen T-Zellen inhibiert, bestehenden Gruppe ausgewählt ist.

10. Mittel zur Verwendung nach Anspruch 9, wobei die Effektorzellen des Patienten nicht expandieren.

11. Mittel zur Verwendung nach Anspruch 9 oder Anspruch 10, wobei die Tumorzellen des Patienten abnehmen.

12. Verwendung nach einem der Ansprüche 4 bis 8 oder Mittel zur Verwendung nach einem der Ansprüche 9 bis 11, wobei es sich bei dem Mittel um Mechlorethamin, Cyclophosphamid, Ifosfamid, Melphalan, Chlorambucil, Hexamethylmelamin, Thiotepa, Busulfan, Carmustin, Lomustin, Semustin, Streptozocin, Dacarbazin, Methotrexat, Fluorurazil, Floxuridin, Cytarabin, Mercaptopurin, Thioguanin, Pentostatin, Vinblastin, Anhydrovinblastin, Vincristin, Etoposid, Teniposid, Dactinomycin, Daunorubicin, Doxorubicin, Bleomycin, Plicamycin, Mitomycin, L-Asparaginase, Cisplatin, Mitoxantron, Hydroxyharnstoff, Procarbazin, Mitotan, Aminoglutethimid, Prednison, Hydroxyprogesteroncaproat, Medroprogesteronacetat, Megestrolacetat, Diethylstilbestrol, Ethinylestradiol, Tamoxifen, Testosteronpropionat, radioaktiven Isotopen, Ricin-A-Kette, Taxol, Diphtherietoxin, Pseudomonas Exotoxin A, einen Anti-CD4-Antikörper, einen Anti-CTLA-4-Antikörper, einen Anti-GITR-Antikörper, einen Anti-CD28-Antikörper oder einen Anti-CD25-Antikörper handelt.

13. Verwendung oder Mittel zur Verwendung nach Anspruch 12, wobei es sich bei dem Mittel um Vinblastin oder Anhydrovinblastin handelt.

## Revendications

1. Procédé pour la détermination du moment où un agent qui inhibe la production de cellules T régulatrices, limite la fonction de celles-ci et/ou détruit celles-ci doit être administré à un sujet mammifère atteint d'un cancer qui a été exposé à un traitement qui augmente le nombre de cellules T effectrices dirigées contre des cellules cancéreuses et/ou active celles-ci, le procédé comprenant l'analyse d'un échantillon obtenu auprès du sujet après le traitement en ce qui concerne les nombres ou l'activité de cellules T effectrices et/ou régulatrices, ou un marqueur inflammatoire de phase aiguë, dans lequel le moment d'administration de l'agent sera choisi de façon à ce qu'il exerce un effet proportionnellement plus grand contre les cellules T régulatrices que contre les cellules T effectrices et dans lequel l'agent est choisi dans le groupe constitué par les médicaments antiprolifératifs, un rayonnement et un anticorps qui inhibe l'activité de régulation négative des cellules T régulatrices.

2. Procédé selon la revendication 1, le procédé comprenant
i) la détermination des nombres de cellules T CD8+CD4- et/ou
ii) la détermination des nombres de cellules tumorales.

3. Procédé selon la revendication 1 ou la revendication 2, le procédé comprenant l'analyse d'échantillons obtenus environ au moins toutes les 48 heures, ou au moins toutes les 24 heures, à partir du sujet après l'augmentation du nombre ou de l'activité de cellules T effectrices.

4. Utilisation d'un agent qui inhibe la production de cellules T régulatrices, limite la fonction de celles-ci et/ou détruit celles-ci pour la fabrication d'un médicament destiné à être administré à un sujet mammifère atteint d'un cancer, dans laquelle l'agent sera administré après que la charge tumorale du sujet a été réduite ce qui permet au nombre et/ou à l' activité de cellules T effectrices, dirigées contre un antigène tumoral, d'augmenter en réponse à une stimulation de cellules T effectrices basée sur un antigène tumoral et dans laquelle le moment d'administration de l'agent sera choisi de façon à ce qu'il exerce un effet proportionnellement plus grand contre les cellules T régulatrices que contre les cellules T effectrices et dans laquelle l'agent est choisi dans le groupe constitué par les médicaments antiprolifératifs, un rayonnement et un anticorps qui inhibe l'activité de régulation négative des cellules T régulatrices.

5. Utilisation d'un composé anticancéreux pour la fabrication d'un médicament destiné à être administré à un sujet mammifère atteint d'un cancer, dans laquelle le composé anticancéreux réduit la charge tumorale ce qui permet au nombre et/ou à l'activité de cellules T effectrices, dirigées contre un antigène tumoral, d'augmenter en réponse à une stimulation de cellules T effectrices basée sur un antigène tumoral et dans laquelle un agent qui inhibe la production de cellules T régulatrices, limite la fonction de celles-ci et/ou détruit celles-ci sera par la suite administré au sujet et dans laquelle le moment d'administration de l'agent sera choisi de façon à ce qu'il exerce un effet proportionnellement plus grand contre les cellules T régulatrices que contre les cellules T effectrices, dans laquelle l'agent est choisi dans le groupe constitué par les médicaments antiprolifératifs, un rayonnement et un anticorps qui inhibe l'activité de régulation négative des cellules T régulatrices et dans laquelle le composé anticancéreux est un médicament antiprolifératif ou un antigène tumoral.

6. Utilisation selon la revendication 4 ou la revendication 5, dans laquelle des fluctuations des niveaux d'un marqueur inflammatoire de phase aiguë chez le sujet seront suivies pour déterminer le moment où l'agent doit être administré.

7. Utilisation selon l'une quelconque des revendications 4 à 6, dans laquelle l'agent sera administré approximativement lorsque
i) le nombre de cellules tumorales a commencé à se stabiliser ou à diminuer à la suite de l'administration de l'antigène tumoral,
ii) le nombre d'antigènes tumoraux circulants a commencé à se stabiliser ou à diminuer à la suite de l'administration de l'antigène tumoral et/ou
iii) les nombres de cellules tumorales sont en train de diminuer et lorsque les nombres de cellules régulatrices sont en train d'augmenter.

8. Utilisation selon l'une quelconque des revendications 4 à 7, dans laquelle le sujet mammifère est un être humain.

9. Agent qui inhibe la production de cellules T régulatrices, limite la fonction de celles-ci et/ou détruit celles-ci destiné à être utilisé en maintien de l'activité et/ou de la production de cellules T effectrices chez un sujet mammifère pour traiter un cancer chez le sujet, **caractérisé en ce que**
(i) la charge tumorale du sujet a été réduite ;
(ii) l'activité et/ou la vitesse de prolifération des cellules T effectrices du sujet ont atteint un pic ; et
(iii) les cellules T régulatrices du sujet sont en train de proliférer,
l'agent étant choisi dans le groupe constitué par les médicaments antiprolifératifs, un rayonnement et un anticorps qui inhibe l'activité de régulation négative des cellules T régulatrices.

10. Agent destiné à être utilisé selon la revendication 9, les cellules effectrices du sujet n'étant pas en train de proliférer.

11. Agent destiné à être utilisé selon la revendication 9 ou la revendication 10, les cellules tumorales du sujet étant en train de diminuer.

12. Utilisation selon l'une quelconque des revendications 4 à 8 ou agent destiné à être utilisé selon l'une quelconque des revendications 9 à 11, l'agent étant la méchloréthamine, le cyclophosphamide, l'ifosfamide, le melphalan, le chlorambucil, l'hexaméthyl-mélamine, le thiotépa, le busulfan, la carmustine, la lomustine, la sémustine, la streptozocine, la dacarbazine, le méthotrexate, le fluorouracile, la floxuridine, la cytarabine, la mercaptopurine, la thioguanine, la pentostatine, la vinblastine, l'anhydrovinblastine, la vincristine, l'étoposide, le téniposide, la dactinomycine, la daunorubicine, la doxorubicine, la bléomycine, la plicamycine, la mitomycine, la L-asparaginase, le cisplatine, la mitoxantrone, l'hydroxyurée, la procarbazine, le mitotane, l'aminoglutéthimide, la prednisone, le caproate d'hydroxyprogestérone, l'acétate de médroprogestérone, l'acétate de mégestrol, le diéthylstilbestrol, l'éthinylestradiol, le tamoxifène, le propionate de testostérone, les isotopes radioactifs, la chaîne A de la ricine, le taxol, la toxine diphtérique, l'exotoxine A de Pseudomonas, un anticorps anti-CD4, un anticorps anti-CTLA-4, un anticorps anti-GITR, un anticorps anti-CD28 ou un anticorps anti-CD25.

13. Utilisation ou agent destiné à être utilisé selon la revendication 12, l'agent étant la vinblastine ou l'anhydrovinblastine.
